# EUROPÄISCHE PATENTSCHRIFT

(11) **EP 1 287 841 B1**
(45) Veröffentlichungstag und Bekanntmachung des Hinweises auf die Patenterteilung: **30.03.2005**
(21) Anmeldenummer: 02007609.7
(22) Anmeldetag: 04.04.2002
(51) Int. Cl.: A61M 5/19, A61M 5/28, A61M 5/315

(54) **Verfahren zur Durchmischung einer schwer löslichen pharmazeutischen Substanz mit einem Lösungsmittel und Spritze zur Anwendung des Verfahrens**
Method for mixing a poorly soluble pharmaceutical substance with a solvent and syringe for use with said method
Procédé pour mélanger une substance pharmaceutique peu soluble avec un solvant et seringue pour la mise en oeuvre de ce procédé

(30) Priorität: 18.08.2001 DE 10140704
(43) Veröffentlichungstag der Anmeldung: 05.03.2003
(73) Patentinhaber: Arzneimittel GmbH Apotheker Vetter & Co. Ravensburg, 88212 Ravensburg (DE)
(72) Erfinder: Vetter, Udo J., 88214 Ravensburg (DE); Otto, Thomas, 88250 Weingarten (DE)
(74) Vertreter: Dziewior, Joachim, Dipl.-Phys. Dr.

(56) Entgegenhaltungen:
- EP-A- 0 793 973
- DE-A- 19 912 322
- US-A- 4 613 326
- US-A- 5 080 649
- US-A- 5 279 608
- US-A- 5 685 846

## Beschreibung

Die Erfindung betrifft ein Verfahren zur Durchmischung einer in vorzugsweise der vorderen, kanülenseitigen Kammer des Spritzenzylinders einer Doppelkammerspritze abgefüllten, schwer löslichen pharmazeutischen Substanz mit einem in der zweiten Kammer abgefüllten Lösungsmittel, wobei beide Kammern durch einen Mittelstopfen voneinander abgetrennt sind. Ferner betrifft die Erfindung eine Spritze zur Anwendung dieses Verfahrens.

Doppelkammerspritzen - wie diese beispielsweise aus der DE 199 12 322 bekannt sind - finden insbesondere dort Anwendung, wo pharmazeutische Substanzen in gelöster Form instabil sind, also relativ schnell verfallen. Daher werden diese Substanzen in trockener, beispielsweise gefriergetrockneter Form in die eine Kammer und das Lösungsmittel getrennt hiervon in die zweite Kammer eingebracht. Vor der Applikation wird dann das Lösungsmittel in die die pharmazeutische Substanz enthaltende Kammer überführt, so daß diese wieder in den gelösten Zustand übergeht. Gegebenenfalls kann die Durchmischung durch Schütteln der Spritze gefördert werden.

Bei solchen Substanzen, die nur schwer wieder in den gelösten Zustand übergehen, ist die auf diese Weise erreichte Durchmischung oftmals nicht ausreichend. Daher muß die Spritze entsprechend lange geschüttelt werden, was nicht nur zeitaufwendig ist, sondern auch die Gefahr von Beschädigungen der Spritze in sich birgt. Andererseits muß jedoch sicher gestellt sein, daß die pharmazeutische Substanz vor der Injektion vollständig aufgelöst ist.

Hiervon ausgehend liegt der Erfindung die Aufgabe zugrunde, ein Verfahren anzugeben, durch das eine schnellere und zuverlässige Durchmischung von pharmazeutischer Substanz und Lösungsmittel erreicht wird. Ferner soll eine Spritze geschaffen werden, durch die dieses Verfahren einfach angewandt werden kann.

In verfahrensmäßiger Hinsicht wird diese Aufgabe in der Weise gelöst, daß zunächst durch Verschiebung des an der Kolbenstange angeordneten Stopfens der Mittelstopfen in den Bereich eines in der Wand des Spritzenzylinders angeordneten Bypasses verschoben und dort an Arretierungsnoppen gehalten wird, worauf dann das Lösungsmittel über den Bypass in die vordere, durch ein kanülenseitig angeordnetes Verschlußsystem abgeschlossene Kammer überführt und hierbei ein Druck in dieser Kammer aufgebaut wird, und daß schließlich der Stopfen unter dem aufgebauten Druck zurückgestellt wird und ein Teil der Lösung gemeinsam mit einem Teil der pharmazeutischen Substanz über den Bypass in die hintere Kammer zurückströmt.

Der durch die Erfindung erreichte Fortschritt besteht im wesentlichen darin, daß durch das mehrfache Durchströmen des Lösungsmittels gemeinsam mit der pharmazeutischen Substanz durch den Bypass hindurch eine starke Verwirbelung erfolgt, durch die ein hohes Maß an Durchmischung erreicht wird. Dies läßt sich auf relativ einfache Weise durch mehrfaches Drücken des Spritzenkolbens in der Art einer pumpenden Betätigung erreichen.

In bevorzugter Ausgestaltung der Erfindung ist vorgesehen, daß der Druckaufbau durch einen lösbaren Anschlag an der Kolbenstange begrenzt wird. Somit ist sichergestellt, daß der Mittelstopfen zunächst nicht über den Bereich des Bypasses hinweg verschoben wird.

Weiter empfiehlt es sich im Rahmen der Erfindung, daß das Überführen und Rückströmen der Lösung mit der pharmazeutischen Substanz dem Grad der Löslichkeit entsprechend häufig wiederholt wird. Dabei kann insbesondere zur Sicherstellung einer vollständigen Auflösung der Substanz eine bestimmte Anzahl von Kolbenhüben vorgeschrieben sein, die vom Anwender einfach durch Mitzählen eingehalten werden kann.

In vorrichtungsmäßiger Hinsicht geht die Erfindung aus von einer Spritze für medizinische Zwecke, bestehend aus einem Spritzenzylinder, wenigstens einem darin angeordneten und mittels einer Kolbenstange verstellbaren Stopfen sowie einer endseitig am Spritzenzylinder angeschlossenen Fingerauflage, die mit einer Durchtrittsöffnung für die Kolbenstange versehen ist, insbesondere in Form einer Doppelkammerspritze, bei der der pharmazeutische Wirkstoff und das Lösungsmittel bis zur Applikation mittels eines weiteren Mittelstopfens voneinander getrennt in einer distalen und einer proximalen Kammer angeordnet sind und über einen Bypass zusammengeführt werden können, insbesondere für pharmazeutische Substanzen mit einer schlechten Löslichkeit.

Die der Erfindung zugrunde liegende Aufgabe wird dadurch gelöst, daß das kanülenseitige Verschlußsystem gegenüber einem im Inneren des Spritzenzylinders sich aufbauenden Druck dichtend und unlösbar fest aufgesetzt ist, daß im Bereich des Bypasses radial nach innen vorstehende Arretierungsnoppen für den Mittelstopfen vorgesehen sind und daß die Kolbenstange mit zwei radial vorstehenden Anschlagelementen versehen ist, wobei das erste, distale Anschlagelement nahe des Stopfens und das zweite, proximale Anschlagelement mit einem solchen Abstand von dem distalen Anschlagelement angeordnet ist, daß bei Anlage dieses Anschlagelements an einer Anschlagleiste der Fingerauflage der Stopfen mit Abstand dem Mittelstopfen gegenübersteht oder ihn allenfalls berührt.

Um einen festen Sitz des Mittelstopfens in beiderlei Strömungsrichtung des Lösungsmittels bzw. der pharmazeutischen Substanz sicherzustellen, ist es zweckmäßig, daß die Arretierungsnoppen den Mittelstopfen in beiderlei Verschiebungsrichtung fixieren.

Nach einer ersten Ausgestaltungsvariante der Erfindung können die Anschlagelemente von radial vorstehenden Gewindezonen und die Anschlagleiste von einem Innengewinde der Fingerauflage gebildet sein. Für die Handhabung vor dem Gebrauch der Spritze ist es dabei weiter von Vorteil, wenn die Gewindezonen sowie das Innengewinde nur wenige Gewindegänge aufweisen, da dann ein schneller Übergang von dem Mischvorgang zur Applikation gegeben ist.

Nach einer zweiten Ausgestaltungsvariante der Erfindung ist vorgesehen, daß die Anschlagelemente von radial vorstehenden Zapfen und die Anschlagleiste von einer radial einwärts vorstehenden Randleiste der Fingerauflage gebildet sind.

Hierzu empfiehlt es sich, daß die Randleiste mit Ausnehmungen zum Durchtritt der Zapfen versehen ist.

Im folgenden wird die Erfindung an einem in der Zeichnung dargestellten Ausführungsbeispiel näher erläutert; es zeigen:
- Fig. 1: eine Doppelkammerspritze im Ausgangszustand, die eine Anwendung des erfindungsgemäßen Verfahrens ermöglicht.
- Fig. 2: die Spritze nach Figur 1 mit in den Bereich des Bypasses verstelltem Mittelstopfen und nahezu vollständig überfülltem Lösungsmittel,
- Fig. 3: die Spritze nach Figur 2, jedoch nach in den Ausgangszustand zurückgestellter Kolbenstange,
- Fig. 4: eine weitgehend gleichartige Darstellung der Figur 2, jedoch mit zum Teil rekonstituierter Substanz,
- Fig. 5: die Spritze gemäß Figur 3, jedoch mit vollständig rekonstituierter Substanz,
- Fig. 6: die Spritze nach Einschrauben der Kolbenstange über die zweite Gewindezone in gebrauchsfertigem Zustand,
- Fig. 7: die Spritze in einer alternativen Ausgestaltung,
- Fig. 8 - 13: die Spritze nach Fig. 7 in den Fig. 1 - 6 entsprechender Funktionsstellung.

Die in der Zeichnung dargestellte Doppelkammerspritze 1 dient in an sich zunächst bekannter Weise dazu, die in der kanülenseitigen Kammer 2 des Spritzenzylinders 3 abgefüllte Substanz in gefriergetrocknetem oder pulverähnlichen Zustand zunächst von dem in der zweiten Kammer 4 abgefüllten Lösungsmittel getrennt zu halten und erst vor dem Gebrauch miteinander zu vermischen. Die Spritze 1 wird dabei in gängiger Technik zusammengesetzt und befüllt, wobei die pharmazeutische Substanz als Pulver abgefüllt oder im Gefriertrockner lyophilisiert wird.

Im einzelnen besteht die Spritze 1 aus dem Spritzenzylinder 3, einem darin angeordneten und mittels einer Kolbenstange 5 verstellbaren Stopfen 6 sowie einer endseitig am Spritzenzylinder 3 angeschlossenen Fingerauflage 7. Diese Fingerauflage 7 ist mit einer Durchtrittsöffnung für die Kolbenstange versehen, die ein Innengewinde aufweist.

Die Doppelkammerspritze ist so ausgebildet, daß bei der pharmazeutische Wirkstoff durch einen Mittelstopfen 8 von dem in der anderen Kammer 4 eingefüllten Lösungsmittel getrennt ist, wobei der Spritzenzylinder 3 mit einem Bypass 9 versehen ist.

Zur Vorbereitung der Applikation des Spritzeninhaltes wird mittels der Kolbenstange 5 der an ihr befestigte Stopfen 6 zum kanülenseitigen Ende hin verstellt, wobei durch den Überdruck im Lösungsmittel der Mittelstopfen 8 zum Bypass 9 hin verschoben wird. Sobald der Mittelstopfen 8 den Weg über den Bypass 9 freigegeben hat, strömt das Lösungsmittel in die kanülenseitige Kammer 2, worauf sich die dort befindliche pharmazeutische Substanz im Lösungsmittel auflösen kann.

Handelt es sich jedoch um eine pharmazeutische Substanz mit einer schlechten Löslichkeit, so müßte anschließend die Spritze beispielsweise so lange geschüttelt werden, bis eine vollständige Auflösung erfolgt ist. Diese Art der Durchmischung ist jedoch vergleichsweise ineffektiv, so daß unter Umständen lange Vorbereitungszeiten notwendig sind, bis die Spritze endlich appliziert werden kann.

Daher weist die Spritze 1 zunächst im Bereich des Bypasses 9 radial nach innen vorstehende Arretierungsnoppen 10 für den Mittelstopfen 8 auf, so daß dieser im Bereich des Bypasses 9 gehalten wird, solange jedenfalls der Stopfen 6 der Kolbenstange 5 ihn nicht über den Bereich des Bypasses 9 hinausschiebt. Ferner ist der Spritzenzylinder 3 durch ein kanülenseitiges Verschlußsystem 11 abgeschlossen, das gegenüber einem im Inneren des Spritzenzylinders 3 sich aufbauenden Druck dichtend und unlösbar fest aufgesetzt ist.

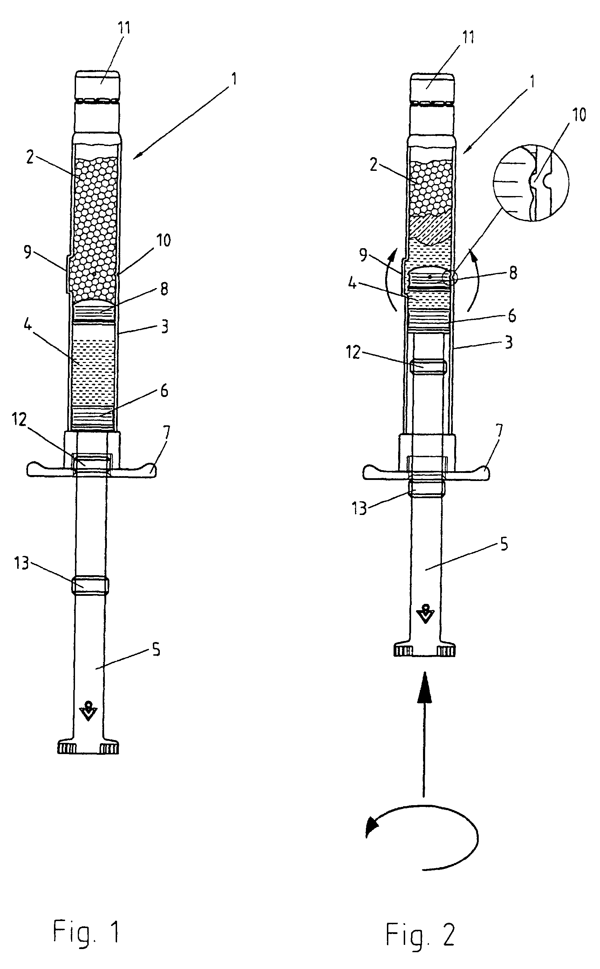

Ferner ist die Kolbenstange 5 in dem Ausführungsbeispiel nach den Fig. 1 bis 6 mit zwei radial vorstehenden Anschlagelementen in Form von Gewindezonen 12,13 versehen, wobei die erste, distale Gewindezone 12 nahe des Stopfens 6 vorgesehen ist und ein unbeabsichtigtes Eindrücken der Kolbenstange 5 verhindert. Zur Vorbereitung der Spritze für die Applikation muß daher zunächst die Gewindezone 12 durch das Innengewinde der Fingerauflage 7 hindurchgeschraubt werden. Die zweite, proximale Gewindezone 13 ist mit einem solchen Abstand von der distalen Gewindezone 12 angeordnet, daß bei Anlage dieser Gewindezone 13 an dem Innengewinde der Fingerauflage 7 der Stopfen 6 mit Abstand dem Mittelstopfen 8 gegenübersteht oder ihn allenfalls berührt. Dadurch ergibt sich beim Eindrücken der Kolbenstange 5 ein Anschlag, der es verhindert, daß der Mittelstopfen 8 über den Bereich des Bypasses 9 hinausgeschoben wird.

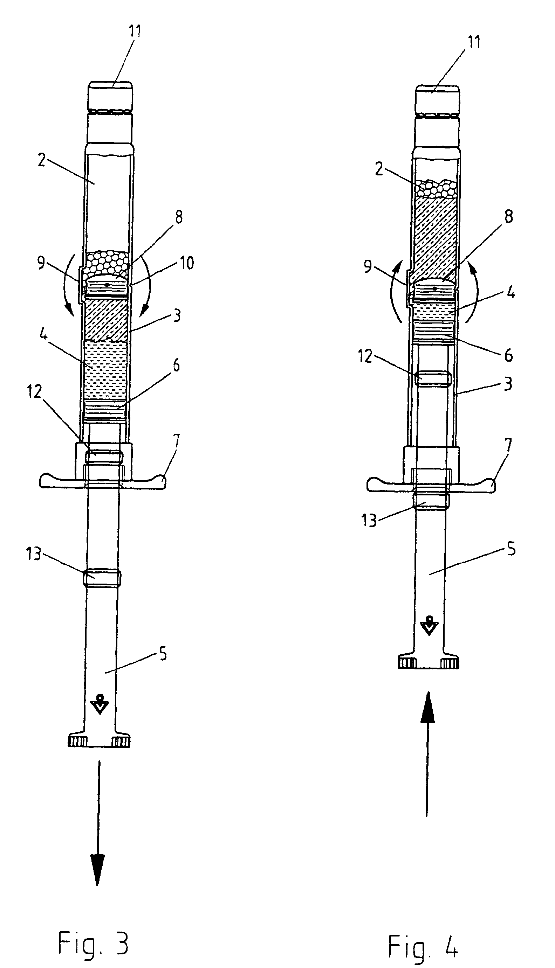

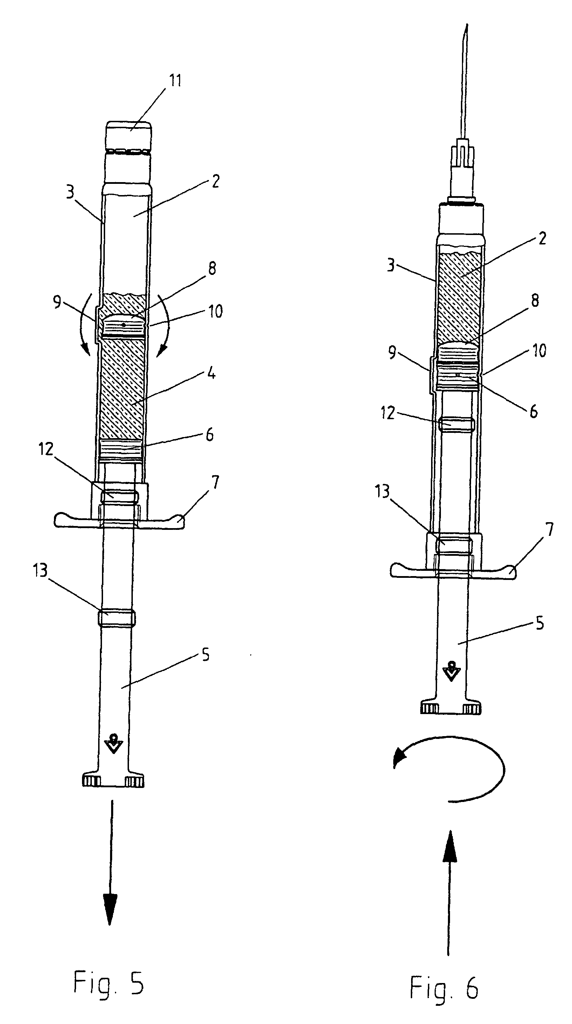

Wird die Kolbenstange 5 zum kanülenseitigen Ende hin verschoben, so baut sich in der vorderen Kammer 2 ein stetig steigender Gegendruck auf. Dieser Gegendruck ist am größten, sobald sich das gesamte Lösungsmittel in der vorderen Kammer 2 befindet und die beiden Stopfen 6,8 sich berühren. Verringert man nun die Kraft auf die Kolbenstange 5, so wird diese unter dem Druck in der Kammer 2 zurückgeschoben mit der Folge, daß das Lösungsmittel zusammen mit der pharmazeutischen Substanz in die zweite Kammer 4 zurückströmt. Danach kann die Kolbenstange 5 erneut betätigt werden, so daß das Lösungsmittel wieder in die kanülenseitige Kammer 2 überführt wird. Dieser Vorgang ist im wesentlichen durch die Figuren 3 und 4 der Zeichnung wiedergegeben.

Das Durchströmen des Bypasses 9, von dem auch mehrere vorhanden sein können, führt zu einer starken Verwirbelung des Lösungsmittels einschließlich der pharmazeutischen Substanz, so daß eine besonders innige Durchmischung und damit eine beschleunigte Auflösung erreicht wird. Durch die Arretierungsnoppen 10 ist sichergestellt, daß der Mittelstopfen 8 gegenüber Druckbelastungen in beiderlei Richtung fixiert ist.

Im Ergebnis erreicht der Anwender durch mehrmaliges Vorschieben und Zurückgleitenlassen der Kolbenstange 5 schließlich eine vollständige Rekonstitution des pharmazeutischen Erzeugnisses.

Das kanülenseitige Verschlußsystem 11 muß so beschaffen sein, daß es den im Inneren des Spritzenzylinders 3 auftretenden Drücken standhält. Dies bedeutet, daß hierfür insbesondere solche Verschlußsysteme in Betracht kommen, die in der Art eines Originalitätsverschlusses ausgebildet sind. Dies bedeutet, daß das Verschlußsystem 11 beispielsweise mit einem Sicherungsring versehen ist, der zunächst entfernt werden muß, ehe beispielsweise ein auf das Nadelansatzstück des Spritzenzylinders 3 aufgesetztes Tip-Cap abgezogen werden kann.

Sobald die vollständige Durchmischung bzw. Lösung der pharmazeutischen Substanz abgeschlossen ist, kann das Verschlußsystem 11 entfernt werden, woraufhin die Kolbenstange 5 mit ihrer zweiten, proximalen Gewindezone 13 in das Innengewinde der Fingerauflage 7 eingeschraubt und hindurchgedreht wird, so daß, sobald sich diese zweite Gewindezone 13 im Inneren des Spritzenzylinders 3 befindet, die Spritze 1 zur Applikation bereit ist.

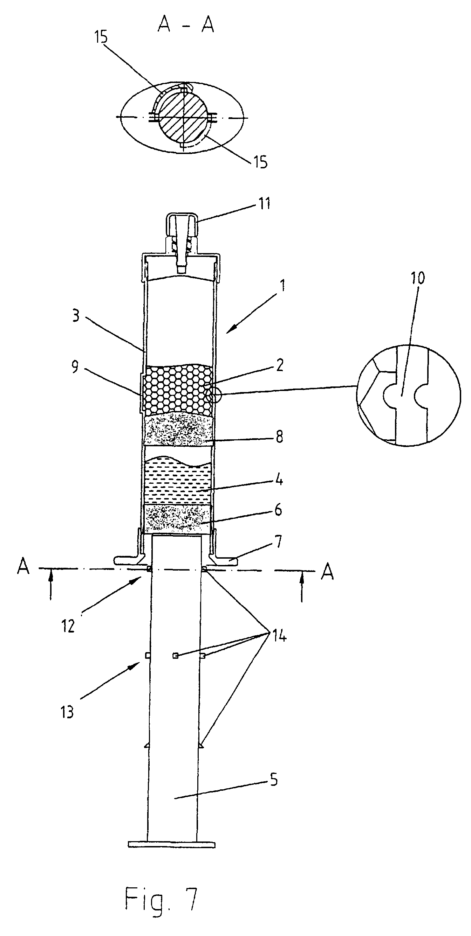

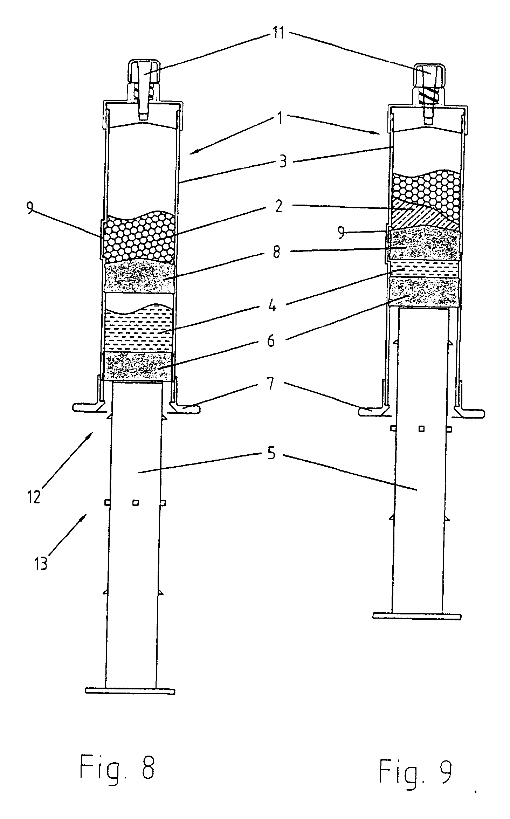

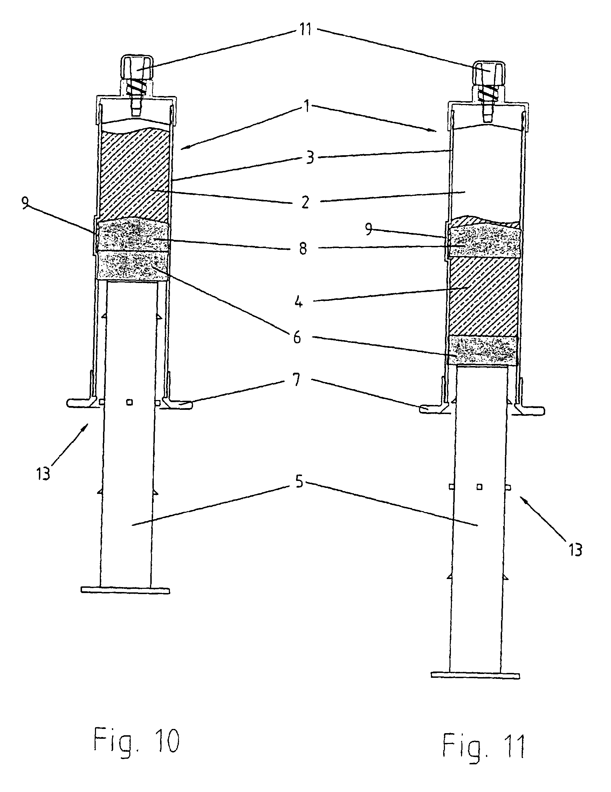

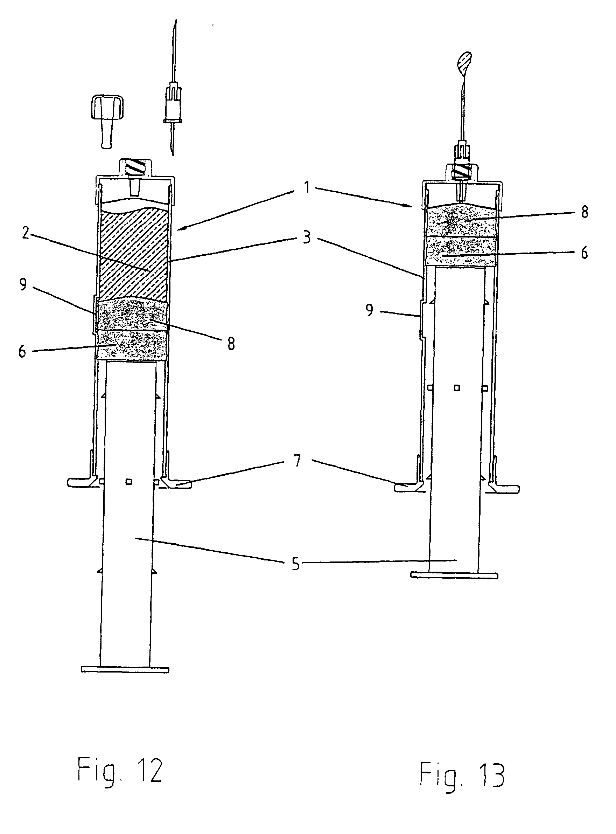

Das weitere Ausführungsbeispiel nach den Fig. 7 bis 13 weist statt der Gewindezonen radial vorstehende Zapfen auf, die entsprechend als Anschlagelemente an einer radial einwärts vorstehenden Randleiste der Fingerauflage wirken. Diese Randleiste ist mit Ausnehmungen versehen, die den Durchtritt der Zapfen nach entsprechender Drehung der Kolbenstange ermöglichen, wie die aus der Querschnittdarstellung der Fig. 7 erkennbar ist. Dadurch ist die Handhabung dieser Ausgestaltung grundsätzlich die gleiche wie die zuvor beschriebene Ausführungsform mit den Gewindezonen.

## Patentansprüche

1. Verfahren zur Durchmischung einer in vorzugsweise der vorderen, kanülenseitigen Kammer (2) des Spritzenzylinders (3) einer Doppelkammerspritze (1) abgefüllten, schwer löslichen pharmazeutischen Substanz mit einem in der zweiten Kammer (4) abgefüllten Lösungsmittel, wobei beide Kammern (2,4) durch einen Mittelstopfen (8) voneinander abgetrennt sind, bei welchem durch Verschiebung des an der Kolbenstange (5) angeordneten Stopfens (6) zunächst der Mittelstopfen (8) in den Bereich eines in der Wand des Spritzenzylinders (3) angeordneten Bypasses (9) verschoben wird, worauf dann das Lösungsmittel über den Bypass (9) in die vordere, durch ein kanülenseitig angeordnetes Verschlußsystem (11) abgeschlossene Kammer (2) überführt wird, **dadurch gekennzeichnet, daß** der Mittelstopfen (8) im Bereich des Bypasses (9) an Arretierungsnoppen (10) gehalten wird und dann ein Druck in der Kammer (2) aufgebaut wird, und daß schließlich der Stopfen (6) unter dem aufgebauten Druck zurückgestellt wird und ein Teil der Lösung gemeinsam mit einem Teil der pharmazeutischen Substanz über den Bypass (9) in die hintere Kammer (4) zurückströmt.

2. Verfahren nach Anspruch 1, **dadurch gekennzeichnet, daß** der Druckaufbau durch einen lösbaren Anschlag an der Kolbenstange (5) begrenzt wird.

3. Verfahren nach Anspruch 1 oder 2, **dadurch gekennzeichnet, daß** das Überführen und Rückströmen der Lösung mit der pharmazeutischen Substanz dem Grad der Löslichkeit entsprechend häufig wiederholt wird.

4. Spritze für medizinische Zwecke zur Anwendung des Verfahrens nach Anspruch 1, mit einem Spritzenzylinder (3), wenigstens einem darin angeordneten und mittels einer Kolbenstange (5) verstellbaren Stopfen (6) sowie einer endseitig am Spritzenzylinder (3) angeschlossenen Fingerauflage (7), die mit einer Durchtrittsöffnung für die Kolbenstange (5) versehen ist, insbesondere in Form einer Doppelkammerspritze, bei der der pharmazeutische Wirkstoff und das Lösungsmittel bis zur Applikation mittels eines weiteren Mittelstopfens (8) voneinander getrennt in einer distalen und einer proximalen Kammer (2,4) angeordnet sind und über einen Bypass (9)zusammengeführt werden können, insbesondere für pharmazeutische Substanzen mit einer schlechten Löslichkeit, **dadurch gekennzeichnet, daß** das kanülenseitige Verschlußsystem (11) gegenüber einem im Inneren des Spritzenzylinders (3) sich aufbauenden Druck dichtend und unlösbar fest aufgesetzt ist, daß im Bereich des Bypasses (9) radial nach innen vorstehende Arretierungsnoppen (10) für den Mittelstopfen (8) vorgesehen sind und daß die Kolbenstange (5) mit zwei radial vorstehenden Anschlagelementen (12,13) versehen ist, wobei das erste, distale Anschlagelement (12) nahe des Stopfens (6) und das zweite, proximale Anschlagelement (13) mit einem solchen Abstand von dem distalen Anschlagelement (12) angeordnet ist, daß bei Anlage dieses Anschlagelements (13) an einer Anschlagleiste der Fingerauflage (7) der Stopfen (6) mit Abstand dem Mittelstopfen (8) gegenübersteht oder ihn allenfalls berührt.

5. Spritze nach Anspruch 4, **dadurch gekennzeichnet, daß** die Arretierungsnoppen (10) den Mittelstopfen (8) in beiderlei Verschiebungsrichtung fixieren.

6. Spritze nach Anspruch 4 oder 5, **dadurch gekennzeichnet, daß** die Anschlagelemente (12,13) von radial vorstehenden Gewindezonen und die Anschlagleiste von einem Innengewinde der Fingerauflage (7) gebildet sind.

7. Spritze nach einem der Ansprüche 4 bis 6, **dadurch gekennzeichnet, daß** die Gewindezonen (12,13) sowie das Innengewinde nur wenige Gewindegänge aufweisen.

8. Spritze nach Anspruch 4 oder 5, dadurch die Anschlagelemente (12,13) von radial vorstehenden Zapfen (14) und die Anschlagleiste von einer radial einwärts vorstehenden Randleiste (15) der Fingerauflage (7) gebildet sind.

9. Spritze nach Anspruch 8, **dadurch gekennzeichnet, daß** die Randleiste (15) mit Ausnehmungen zum Durchtritt der Zapfen (14) versehen ist.

## Claims

1. A method of mixing a poorly soluble pharmaceutical substance contained in preferably the front, cannula-end chamber (2) of the syringe cylinder (3) of a double-chamber syringe (1) with a solvent contained in the second chamber (4), wherein the two chambers (2, 4) are separated from each other by a central plunger (8), wherein by displacement of the plunger (6) arranged on the piston rod (5) firstly the central plunger (8) is moved into the region of a bypass (9) arranged in the wall of the syringe cylinder (3), whereupon then the solvent is transferred by way of the bypass (9) into the front chamber (2) which is closed off by a closure system (11) arranged at the cannula end, **characterised in that** the central plunger (8) is stopped at arresting knobs (10) in the region of the bypass (9) and then a pressure is built up in the chamber (2), and finally the plunger (6) is returned under the pressure which is built up and a part of the solution jointly with a part of the pharmaceutical substance flows back by way of the bypass (9) into the rear chamber (4).

2. A method according to claim 1 **characterised in that** the pressure build-up is limited by a releasable abutment on the piston rod (5).

3. A method according to claim 1 or claim 2 **characterised in that** transfer and return flow of the solution with the pharmaceutical substance is repeated frequently according to the degree of solubility.

4. A syringe for medical purposes for using the method according to claim 1, comprising a syringe cylinder (3), at least one plunger (6) arranged therein and displaceable by means of a piston rod (5) and a finger support (7) connected at the end to the syringe cylinder (3) and provided with a through opening for the piston rod (5), in particular in the form of a double-chamber syringe, wherein until application the pharmaceutical active substance and the solvent are arranged separately from each other by means of a further central plunger (8) in a distal and a proximal chamber (2, 4) and can be brought together by way of a bypass (9), in particular for pharmaceutical substances involving poor solubility, **characterised in that** the cannula-end closure system (11) is fitted on non-releasably firmly and sealingly in relation to a pressure which builds up in the interior of the syringe cylinder (3), that provided in the region of the bypass (9) are radially inwardly projecting arresting knobs (10) for the central plunger (8) and that the piston rod (5) is provided with two radially projecting abutment elements (12), wherein the first distal abutment element (12) is arranged near the plunger (6) and the second proximal abutment element (13) is arranged at such a spacing from the distal abutment element (12) that when said abutment element (13) bears against an abutment bar of the finger support the plunger (6) is in opposite relationship at a spacing with respect to the central plunger (8) or at most touches it.

5. A syringe according to claim 4 **characterised in that** the arresting knobs (10) fix the central plunger (8) in both directions of displacement.

6. A syringe according to claim 4 or claim 5 **characterised in that** the abutment elements (12, 13) are formed by radially projecting screwthread zones and the abutment bar is formed by a female screwthread of the finger support (7).

7. A syringe according to one of claims 4 to 6 **characterised in that** the screwthread zones (12, 133) and the female screwthread have only few screwthread pitches.

8. A syringe according to claim 4 or claim 5 **characterised in that** the abutment elements (12, 13) are formed by radially projecting pegs (14) and the abutment bar is formed by a radially inwardly projecting edge bar (15) of the finger support (7).

9. A syringe according to claim 8 **characterised in that** the edge bar (15) is provided with openings for the pegs (14) to pass therethrough.

## Revendications

1. Procédé de mélange d'une substance pharmaceutique difficilement soluble, contenue dans la chambre (2) antérieure, côté canule, du corps (3) d'une seringue à chambre double (1) avec un solvant contenu dans la seconde chambre (4), les deux chambres (2, 4) étant séparées l'une de l'autre par un piston intermédiaire (8), selon lequel suite au déplacement du piston (6) lié à la tige de piston (8), tout d'abord le piston intermédiaire (8) est déplacé dans la zone d'un bypass (9) aménagé dans la paroi du corps de seringue (3) et y est retenu par des saillies d'arrêt (10), puis le solvant est transféré via le bypass (9) dans la chambre (2) antérieure, fermée par un système de fermeture (11) prévu côté canule, et une pression est établie dans ladite chambre (2), et enfin le piston (6) est repoussé par l'action de la pression établie et une partie de la substance pharmaceutique reflue à travers le bypass (9), dans la chambre postérieure (4).

2. Procédé selon la revendication 1, **caractérisé par le fait que** la montée en pression est limitée par une butée déclenchable sur la tige de piston (5).

3. Procédé selon la revendication 1 ou 2, **caractérisé par le fait que** le transfert et le reflux de la solution contenant la substance pharmaceutique sont répétés autant de fois que l'exige le degré de solubilité.

4. Seringue à usage médical pour la mise en oeuvre du procédé selon la revendication 1, comportant au moins un corps de seringue (3), au moins un piston (6) disposé à l'intérieur de celui-ci et déplaçable au moyen d'une tige de piston (5), ainsi qu'une collerette repose-doigt (7) qui est liée à l'extrémité du corps de seringue (3) et est pourvue d'une ouverture de passage pour la tige de piston, en particulier sous la forme d'une seringue à chambre double, dans laquelle la substance pharmaceutique et le solvant sont contenus en étant séparés l'un de l'autre par un piston intermédiaire (8) jusqu'à l'instant de l'administration dans une chambre distale et une chambre proximale et peuvent être réunis par l'intermédiaire d'un bypass (9), notamment pour des substances pharmaceutiques difficilement solubles, **caractérisée par le fait que** le système de fermeture (11) côté canule est monté de manière non séparable et étanche vis-à-vis d'une pression qui s'établit à l'intérieur du corps de seringue (3), qu'il est prévu dans la région du bypass (9) des saillies d'arrêt (10) du piston intermédiaire (8) saillantes radialement en direction de l'intérieur, **par le fait que** la tige de piston (5) est pourvue de deux éléments de butée (12, 13) saillants radialement, le premier élément de butée (12) distal étant disposé dans le voisinage du piston (6) et le second élément de butée (13) proximal étant disposé à une distance de l'élément de butée (12) distal telle, que lorsque ledit élément de butée (13) est en contact avec une nervure formant butée sur la collerette repose-doigts (7) le piston (6) soit écarté du piston intermédiaire (8) ou au plus le touche.

5. Seringue selon la revendication 4, **caractérisée par le fait que** les saillies d'arrêt (10) bloquent le piston intermédiaire (8) dans les deux directions de déplacement.

6. Seringue selon la revendication 4 ou 5, **caractérisée par le fait que** les éléments de butée (12, 13) sont formés pas des zones filetées saillantes radialement et que la nervure de butée est formée par un filetage intérieur de la collerette repose-doigts (7).

7. Seringue selon une des revendications 4 à 6, **caractérisée par le fait que** les zones filetées (12, 13) ainsi que le filetage intérieur ne présentent que quelques filets.

8. Seringue selon la revendication 4 ou 5, **caractérisée par le fait que** les éléments de butée (12, 13) sont formés pas des tétons saillants radialement et que la nervure de butée est formée par une nervure périphérique (15) saillante radialement en direction de la collerette repose-doigts (7).

9. Seringue selon la revendication 8, **caractérisée par le fait que** la nervure périphérique (15) est pourvue d'échancrures pour le passage des tétons (14).
